# EUROPÄISCHE PATENTANMELDUNG

(11) **EP 4 606 764 A1**
(43) Veröffentlichungstag der Anmeldung: **27.08.2025**
(21) Anmeldenummer: 24020063.4
(22) Anmeldetag: 23.02.2024
(51) Int. Cl.: C01B 3/32, C07C 29/151

(54) **BEREITSTELLUNG UND REFORMIERUNG VON METHANOL**

(71) Anmelder: Linde GmbH, 82049 Pullach (DE); Technische Universität München, 80333 München (DE)
(72) Erfinder: Dillig, Marius, 82049 Pullach (DE); Haselsteiner, Thomas, 82049 Pullach (DE); Klein, Harald, 80290 München (DE); Fahr, Steffan, 80290 München (DE)
(74) Vertreter: Fischer, Werner

(57) **Zusammenfassung**

Es wird ein Verfahren (100) zur Reformierung von Methanol vorgeschlagen, bei dem ein Wasser und Methanol enthaltendes Reformierungseinsatzgemisch (14) bereitgestellt und einem oder mehreren Reformierungsschritten (120) unterworfen wird, wobei die Bereitstellung des Reformierungseinsatzgemischs (14) einen oder mehrere Methanolsyntheseschritte umfasst, in dem oder denen ein Wasser und Methanol enthaltendes Methanolsyntheseproduktgemisch (7) gebildet wird, wobei das in dem Methanolsyntheseproduktgemisch (7) enthaltene Wasser oder ein Teil hiervon und das in dem Methanolsyntheseproduktgemisch (7) enthaltene Methanol oder ein Teil hiervon ohne eine Trennung voneinander in das Reformierungseinsatzgemisch (14) überführt werden. Eine entsprechende Anlage wird ebenfalls vorgeschlagen.

## Beschreibung

Die Erfindung betrifft das Gebiet der Bereitstellung und Reformierung von Methanol.

### Hintergrund

Verfahren zur Herstellung von Wasserstoff beruhen herkömmlicherweise auf der Umsetzung von kohlen(wasser)stoffhaltigen Einsätzen oder der Elektrolyse von Wasser unter Verwendung an sich bekannter Verfahren. Ein weiterer Weg zur Herstellung von Wasserstoff ist die Reformierung von Methanol.

Bei Methanol handelt es sich aufgrund seiner Energiedichte und einfachen Handhabung um einen besonders vorteilhaften Wasserstoffträger. Insbesondere dann, wenn die Herstellung von Methanol unter Einsatz von erneuerbarer Energieen, d.h. wenn es scih um sogenanntes grünes Methanol handelt, ist die Verwendung besonders emissionsarm. Der durch die Reformierung von Methanol gewonnene Wasserstoff kann beispielsweise mit Hilfe einer Brennstoffzelle in Strom umgewandelt werden.

### Überblick

Es werden Verfahren und Anlagen mit den jeweiligen Merkmalen der unabhängigen Ansprüche vorgeschlagen. Ausgestaltungen sind Gegenstand der abhängigen Ansprüche und der nachfolgenden Beschreibung.

Das vorgeschlagene Verfahren zur Bereitstellung und Reformierung von Methanol umfasst, dass ein Wasser und Methanol enthaltendes Reformierungseinsatzgemisch bereitgestellt und einem oder mehreren Reformierungsschritten unterworfen wird, wobei die Bereitstellung des Reformierungseinsatzgemischs einen oder mehrere Methanolsyntheseschritte umfasst, in dem oder denen ein Wasser und Methanol enthaltendes Methanolsyntheseproduktgemisch gebildet wird, wobei das in dem Methanolsyntheseproduktgemisch enthaltene Wasser oder ein Teil hiervon und das in dem Methanolsyntheseproduktgemisch enthaltene Methanol oder ein Teil hiervon ohne eine Trennung voneinander in das Reformierungseinsatzgemisch überführt werden.

Das vorgeschlagene Verfahren umfasst also, dass ein Gemisch aus Wasser und Methanol, das hier als Reformierungseinsatzgemisch bezeichnet wird, aus Kohlendioxid und Wasser hergestellt wird. Dieses kann insbesondere für eine bestimmte Zeit gespeichert und/oder transportiert werden. Ferner umfasst das vorgeschlagene Verfahren, dieses Gemisch als Ausgangsmaterial für die Methanolreformierung zu verwenden. Das Gemisch kann insbesondere hergestellt werden aus dem Reaktionsprodukt der direkten Hydrierung von Kohlendioxid mit minimaler oder sogar ohne Reinigung und einer optionalen Zugabe kleiner Wassermengen, um das gewünschte Verhältnis von Wasser zu Methanol für die Reformierung zu erreichen.

In bestimmten Ausgestaltungen kann eine Aufbereitung des Methanolsyntheseproduktgemischs oder eines Teils hiervon vorgesehen sein. Die Aufbereitung kann einer herkömmlichen Aufbereitung eines Methanolsyntheseproduktgemischs entsprechen. Daher kann hier auf bewährte Verfahren zurückgegriffen werden. Insbesondere erfolgt dabei jedoch keine Trennung von Wasser und Methanol voneinander.

In bestimmten Ausgestaltungen kann die Aufbereitung des Methanolsyntheseproduktgemischs oder von dessen Teil eine Bildung eines Zwischengemischs umfassen, wobei das Zwischengemisch mehr als 99% Wasser und Methanol in Summe enthält. Dieses Zwischengemisch muss in hier vorgeschlagenen Ausgestaltungen insbesondere nicht oder nur geringfügig für die Methanolreformierung bearbeitet werden.

In Ausgestaltungen, in denen eine Bearbeitung des Zwischengemischs vorgesehen ist, kann diese als eine nachfolgend erläuterte Konditionierung des Zwischengemischs oder eines Teils hiervon durchgeführt werden. Dies kann zu einer noch besseren Anpassung an die jeweiligen Erfodernisse führen.

Beispielsweise kann die Konditionierung eine Einstellung eines Zielverhältnisses von Wasser zu Methanol durch eine Zuspeisung von Wasser umfassen, um dieses insbesondere in einen Bereich um das stöchiometrische Verhältnis von Methanol zu Wasser von ca. 36:64 zu bringen.

Das Zielverhältnis von Wasser zu Methanol kann in Ausgestaltungen insbesondere zwischen 20:80 und 80:40, zwischen 30:70 und 45:55 oder zwischen 30:70 und 45:55 Molanteilen betragen..

Eine Konditionierung kann in Ausgestaltungen der Erfindung insbesondere auch eine Entfernung einer oder mehrerer leichtflüchtiger Verbindungen umfassen.

Die eine oder die mehreren leichtflüchtigen Verbindungen können aus Kohlendioxid, einem oder mehreren Estern, einem oder mehreren Ethern, einem oder mehreren Aldehyden und/oder einem oder mehreren Ketonen ausgewählt sein.

In Ausgestaltungen kann die Entfernung der einen oder der mehreren leichtflüchtigen Verbindungen unter Einsatz einer Rektifikation durchgeführt werden.

In bestimmten Ausgestaltungen kann die Entfernung der einen oder der mehreren leichtflüchtigen Verbindungen zusätzlich oder alternativ dazu aber auch unter Einsatz einer Strippung durchgeführt werden.

Die Strippung kann, wie insoweit typisch, unter Verwendung von Strippgas und unter Erhalt eines Strippabgases durchgeführt wird.

In bestimmten Ausgestaltungen kann das Strippgas Wasserstoff enthalten und das Strippabgas oder ein Teil hiervon kann in das Verfahren zurückgeführt werden.

In bestimmten Ausgestaltungen kann zusätzlich oder alternativ dazu das Strippgas aber auch Stickstoff enthalten und das Strippabgas oder ein Teil hiervon kann aus dem Verfahren ausgeleitet werden.

Die Aufbereitung kann nacheinander eine erste Trennung auf einem ersten Druckniveau, eine Entspannung auf ein zweites Druckniveau und eine zweite Trennung auf dem zweiten Druckniveau umfasst.

Das vorgeschlagene Verfahren zur Bereitstellung eines Reformierungseinsatzgemischs umfasst, einen oder mehrere Methanolsyntheseschritte durchzuführen, in dem oder denen ein Wasser und Methanol enthaltendes Methanolsyntheseproduktgemisch gebildet wird, wobei das in dem Methanolsyntheseproduktgemisch enthaltene Wasser oder ein Teil hiervon und das in dem Methanolsyntheseproduktgemisch enthaltene Methanol oder ein Teil hiervon ohne eine Trennung voneinander in das Reformierungseinsatzgemisch überführt werden und das Reformierungseinsatzgemisch dem Verfahren entzogen und einer Speicherung und/oder einem Transport unterworfen wird. Hinsichtlich der Bereitstelluing des Reformierungseinsatzgemischs können dabei sämtliche zuvor erläuterte Ausgestaltungen zum Einsatz kommen.

Die vorgeschlagene Anlage zur Bereitstellung und Reformierung von Methanol ist dafür eingerichtet, ein Wasser und Methanol enthaltendes Reformierungseinsatzgemisch bereitzustellen und einem oder mehreren Reformierungsschritten zu unterwerfen, wobei die Anlage zur Bereitstellung des Reformierungseinsatzgemischs durch einen oder mehrere Methanolsyntheseschritte eingerichtet ist, in dem oder denen ein Wasser und Methanol enthaltendes Methanolsyntheseproduktgemisch gebildet wird, und wobei die Anlage dafür eingerichtet ist, das in dem Methanolsyntheseproduktgemisch enthaltene Wasser oder ein Teil hiervon und das in dem Methanolsyntheseproduktgemisch enthaltene Methanol oder ein Teil hiervon ohne eine Trennung voneinander in das Reformierungseinsatzgemisch überführt werden.

Die vorgeschlagene Anlage zur Bereitstellung eines Reformierungseinsatzgemischs ist dafür eingerichtet, einen oder mehrere Methanolsyntheseschritte durchzuführen, in dem oder denen ein Wasser und Methanol enthaltendes Methanolsyntheseproduktgemisch gebildet wird, wobei die Anlage dafür eingerichtet ist, das in dem Methanolsyntheseproduktgemisch enthaltene Wasser oder einen Teil hiervon und das in dem Methanolsyntheseproduktgemisch enthaltene Methanol oder einen Teil hiervon ohne eine Trennung voneinander in das Reformierungseinsatzgemisch zu überführen und das Reformierungseinsatzgemisch für eine Speicherung und/oder einen Transport aus der Anlage auszuschleusen.

Zu weiteren Merkmalen und Vorteilen entsprechender Anlage und Ausgestaltungen hiervon sei auf die obigen Erläuterungen betreffend die vorgeschlagenen Verfahren und ihre Ausgestaltungen verwiesen, da diese hierfür in gleicher Weise gelten.

Entsprechendes gilt auch für Anlagen, die gemäß Ausgestaltungen dazu eingerichtet sind, Verfahren gemäß beliebiger Ausgestaltungen durchzuführen.

### Zeichnungen

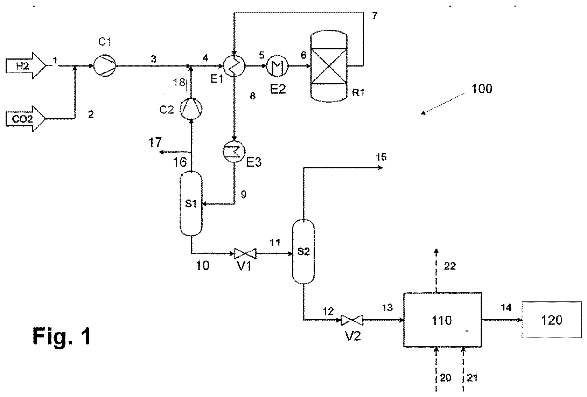

Ausführungsformen der Erfindung werden nachfolgend rein beispielhaft unter Bezugnahme auf die beigefügte Zeichnung beschrieben, wobei
Figur 1 ein Verfahren gemäß einer vorgeschlagenen Ausgestaltung veranschaulicht.

### Ausführungsformen

Die nachfolgend beschriebenen Ausführungsformen werden lediglich zu dem Zweck beschrieben, den Leser beim Verständnis der beanspruchten und zuvor erläuterten Merkmale zu unterstützen. Sie stellen lediglich Beispiele dar und sollen hinsichtlich der Merkmale der Erfindung nicht abschließend und/oder beschränkend verstanden werden. Es versteht sich, dass die zuvor und nachfolgend beschriebenen Vorteile, Ausführungsformen, Beispiele, Funktionen, Merkmale, Strukturen und/oder anderen Aspekte nicht als Beschränkungen des Umfangs der Erfindung, wie er, wie zuvor erwähnt, in den Ansprüchen definiert ist, oder als Beschränkungen von Äquivalenten zu den Ansprüchen zu betrachten sind, und dass andere Ausführungsformen verwendet und Änderungen vorgenommen werden können, ohne vom Umfang der beanspruchten Erfindung abzuweichen.

Unterschiedliche Ausführungsformen der Erfindung können weitere zweckmäßige Kombinationen der beschriebenen Elemente, Komponenten, Merkmale, Teile, Schritte, Mittel usw. umfassen, aufweisen, aus ihnen bestehen oder im Wesentlichen aus ihnen bestehen, auch wenn solche Kombinationen hier nicht spezifisch beschrieben sind. Darüber hinaus kann die Offenbarung andere Erfindungen umfassen, die gegenwärtig nicht beansprucht sind, die aber in Zukunft beansprucht werden können, insbesondere wenn sie vom Umfang der unabhängigen Ansprüche umfasst sind.

Erläuterungen, die sich auf Vorrichtungen, Apparate, Anordnungen, Systeme usw. gemäß Ausführungsformen der vorliegenden Erfindung beziehen, können auch für Verfahren, Prozesse, Methoden usw. gemäß den Ausführungsformen der vorliegenden Erfindung gelten und umgekehrt. Gleiche, gleich wirkende, in ihrer Funktion einander entsprechende, baulich identisch oder vergleichbar aufgebaute Elemente, Verfahrensschritte usw. können mit identischen Bezugszeichen angegeben sein.

Verfahren zur Herstellung von Wasserstoff, die im Zusammenhang mit der vorliegenden Erfindung zum Einsatz kommen können, sind vielfach in der Literatur beschrieben. Statt vieler sei in diesem Zusammenhang beispielsweise auf den Artikel von A.O. Oni et al., "Comparative assessment of blue hydrogen from steam methane reforming, autothermal reforming, and natural gas decomposition technologies for natural gas-producing regions", Energy Conversion and Management 254 (2022) 115245, verwiesen, der in den Figuren 2 bis 4 solche Verfahren zeigt und in den jeweils zugehörigen Textpassagen beschreibt.

Die Herstellung von Wasserstoff mittels Wasserelektrolyse ist ebenfalls hinreichend bekannt und beispielsweise in dem Artikel "Hydrogen" in Ullmann's Encyclopedia of Industrial Chemistry, Wiley-VCH, 15. Juni 2000, DOI: 10.1002/14356007.a13_297, insbesondere in Abschnitt 4.2, "Electrolysis", beschrieben.

Bei der klassischen Wasserelektrolyse wird eine wässrige alkalische Lösung, typischerweise von Kaliumhydroxid, als Elektrolyt verwendet (AEL, Alkalische Elektrolyse). Die Elektrolyse mit einer uni- oder bipolaren Elektrodenanordnung erfolgt dabei bei Atmosphärendruck oder im industriellen Maßstab auch deutlich darüber. Neuere Entwicklungen bei der Wasserelektrolyse umfassen die Verwendung von protonenleitenden lonenaustauschmembranen (SPE, Solid Polymer Electrolysis; PEM, Proton Exchange Membranes), bei der das zu elektrolysierende Wasser an der Anodenseite bereitgestellt wird. Auch die Elektrolyse unter Verwendung von Anionenaustauschermembranen (AEM, Anion Exchange Membrane) ist bekannt.

Die bisher genannten Verfahren der Wasserelektrolyse zählen zu den Niedertemperaturverfahren, bei denen das zu elektrolysierende Wasser in der Flüssigphase vorliegt. Daneben wird auch die sogenannte Dampfelektrolyse eingesetzt, die ebenfalls mit alkalischen Elektrolyten (also als AEL) mit angepassten Membranen, beispielsweise Polysulfonmembranen, sowie unter Verwendung von Festoxidelektrolysezellen (SOEC, Solid Oxide Electrolysis Cells) durchgeführt werden können. Letztere umfassen insbesondere dotiertes Zirkondioxid oder Oxide anderer seltener Erden, die bei mehr hohen Temperaturen leitfähig werden.

Der Begriff der Elektrolyse soll nachfolgend sämtliche dieser Verfahren umfassen. Insbesondere die Niedertemperaturelektrolyse (PEM, AEL, AEM) eignet sich für einen flexiblen Betrieb, der den Energieübergang zu erneuerbaren Energien unterstützt. Sämtliche Verfahren können in den hier vorgeschlagenen Verfahren und entsprechenden Ausgestaltungen eingesetzt werden. Für eine weitere Übersicht wird auf Fachliteratur wie S. Mucci et al., "Power-to-Xprocesses based on PEM water electrolyzers: A review of process integration and flexible operation", Comput. & Chem. Eng. 175, 2023, 108260, verwiesen.

Die Grundprinzipien der Methanolsynthese und Methanolreformierung wurden bereits eingangs erläutert. Weitere Details sind ebenfalls in entsprechender Fachliteratur, wie beispielsweise D.R. Palo et al., "Methanol Steam Reforming for Hydrogen Production" Chem. Rev. 107, 2007, 3992, M.S. Herdem et al., "An overview of the methanol reforming process: Comparison of fuels, catalysts, reformers, and systems." Energy Res. 43, 2019, 5076-5105, sowie im Artikel "Methanol" in Ullmann's Encyclopedia of Industrial Chemistry und Patentliteratur wie der US 4,210,495 A beschrieben.

Flüssige und gasförmige Stoffströme, Gasgemische oder dergleichen können im hier verwendeten Sprachgebrauch "reich" oder "arm" an einer oder mehreren Komponenten sein, wobei "reich" für einen Gehalt von wenigstens 50%, 75%, 90%, 95%, 99%, 99,5%, 99,9% oder 99,99% und "arm" für einen Gehalt von höchstens 50%, 25%, 10%, 5%, 1%, 0,1% oder 0,01% auf molarer, Gewichts- oder Volumenbasis stehen kann.

Flüssige und gasförmige Stoffströme, Gasgemische oder dergleichen können im hier verwendeten Sprachgebrauch ferner angereichert oder abgereichert an einer oder mehreren Komponenten sein, wobei sich diese Begriffe auf einen Gehalt in einem anderen Stoffstrom beziehen, unter Verwendung dessen der Stoffstrom gebildet wurde. Ein betrachteter Stoffstrom ist dabei "angereichert", wenn er zumindest den 2-fachen, 5-fachen, 10-fachen, 100-fachen oder 1.000-fachen Gehalt der bezeichneten Komponente(n) aufweist, und "abgereichert", wenn er höchstens den 0,5-fachen, 0,1-fachen, 0,01-fachen oder 0,001-fachen Gehalt der bezeichneten Komponente(n) aufweist, jeweils in Bezug auf den Stoffstrom, unter Verwendung dessen der betrachtete Stoffstrom gebildet wurde.

Unter Angaben wie "im Wesentlichen enthaltend" und dergleichen soll hier insbesondere verstanden werden, dass in einer damit beschriebenen Zusammensetzung, einem Stoffstrom usw. neben den als obligatorisch angegebenen bzw. sich aus der Bezeichnung des Gasgemischs (bspw. "Wasserstoff) ergebenden obligatorischen Bestandteilen weitere Komponenten enthalten sein können, sofern sich die wesentlichen Merkmale der damit bezeichneten Zusammensetzung durch diese nicht signifikant ändern. Entsprechendes gilt auch Angaben wie "im Wesentlichen frei von" und dergleichen. Ein "im Wesentlichen" eine oder mehrere Komponenten enthaltendes oder aus diesen bestehendes Gas bzw. Gasgemisch kann insbesondere diese Komponenten zu mehr als 95, 99, 99,9 oder 99,99% in Summe oder als Einzelwerte aufweisen. Umgekehrt ist ein Gas bzw. Gasgemisch "im Wesentlichen frei" von einer oder mehreren Komponenten, wenn es weniger als 5, 1, 0,1 oder 0,01% dieser Komponenten in Summe oder als Einzelwerte enthält.

Sämtliche hier verwendeten Prozentangaben können sich auf Mol-, Mengen- oder Volumenanteile beziehen. Druckangaben in bar sind, soweit nicht anders erläutert, insbesondere als Absolutdrücke zu verstehen.

Die Konjunktion "und/oder" soll, wenn in einer Aufzählung vor dem letzten Begriff der Aufzählung verwendet, so verstanden werden, dass alle in der Aufzählung zuvor genannten Begriffe in beliebiger Weise miteinander kombiniert werden können. Mit anderen Worten ist mit "A, B und/oder C" "A und/oder B und/oder C" oder "wenigstens eines der Elemente A, B und C in beliebiger Kombination" gemeint.

Ist vorliegend von einem "Teil" eines Stoffstroms die Rede, kann es sich hierbei um einen Mengenanteil mit gleicher Zusammensetzung handeln, der von einem Ausgangsstrom lediglich abgezweigt wurde, aber auch um einen Anteil abweichender Zusammensetzung und ggf. nur eine Komponente des Ausgangsstroms, der mittels eines Verfahrens wie Kondensation, Evaporation, Sieden, Destillieren, Rektifizieren, Absorbieren, Absorbieren, Flashen, Membrantrennen, Abscheiden oder dergleichen gebildet wird oder bei einem entsprechenden Schritt als Rest verbleibt. Ein "Teil" kann auch nach einer Kombination beliebiger der vorstehend benannten Schritte vorliegen, beispielsweise nach trenntechnischer Bearbeitung abgezweigten Anteils.

Die Methanolerzeugung im industriellen Maßstab basiert auf der Umsetzung von Wasserstoff und Kohlenmonoxid und/oder Kohlendioxid an einem Katalysator. Da die Umwandlung des Reaktoreinsatzes pro Durchgang durch das Reaktionsgleichgewicht begrenzt ist, wird ein großer Teil der Edukte in den Reaktionseinsatz zurückgeführt, nachdem ein Teil der Reaktionsprodukte auskondensiert und entfernt wurde.

Die herkömmliche Methanolsynthese beruht auf der Herstellung der Edukte aus fossilen Einsatzstoffen, beispielsweise mittels Dampfreformierung, Autothermreformierung oder Partialoxidation von Kohlenwasserstoffen (z.B. Erdgas, Naphtha usw.) oder der Kohlevergasung oder einer Kombination solcher Verfahren. Das Einsatzgas einer herkömmlichen Methanolsynthese besteht hauptsächlich aus Wasserstoff und Kohlenmonoxid.

In jüngster Zeit hat sich der industrielle Schwerpunkt auf die Herstellung von sogenanntem grünem Methanol aus durch Elektrolyse gewonnenem Wasserstoff und Kohlendioxid verlagert. Vorgeschlagen wurden in diesem Zusammenhang sowohl die direkte Hydrierung von Kohlendioxid als auch die Umwandlung von Kohlendioxid in Kohlenmonoxid in einer reversen Wassergasshift oder eine Kohlendioxidelektrolyse mit anschließendem konventionellem Synthesekreislauf. Die direkte Hydrierung kann aufgrund ihrer geringeren Prozesskomplexität von Vorteil sein.

Die Gesamtreaktion, die bei der direkten Hydrierung von Kohlendioxid abläuft, lautet

CO₂ + 3 H₂ → CH₃OH + H₂O.

Bei beiden Verfahrensvarianten erfolgt in der Regel die Reinigung des Methanolprodukts in zwei bis drei Destillationskolonnen. Die Reinigung bei der direkten Hydrierung weicht aufgrund der erhöhten Wasserproduktion im Reaktor geringfügig ab und kann energieintensiver sein. Nichtsdestotrotz ist die Reinigung bei beiden Verfahren energieintensiv und kann ein schlechtes dynamisches Verhalten und eine geringe Teillastfähigkeit aufweisen. Dies kann insbesondere dann nachteilig sein, wenn schwankende erneuerbare Energiequellen für die Wasserstoffproduktion und/oder den Betrieb der übrigen Prozessausrüstung, wie z. B. der Kolonnen selbst, genutzt werden.

Die Methanolreformierung erfolgt unter wesentlich milderen Bedingungen als die Reformierung von Kohlenwasserstoffen, wie z.B. die Dampfreformierung von Methan, und eignet sich daher gut für kleine und sogar mobile Anwendungen. Die Methanolreformierung ist im Wesentlichen der umgekehrte Prozess der Methanolsynthese und läuft unter ähnlichen Prozessbedingungen und mit ähnlichen Katalysatoren ab. Die Reaktionsgleichung lautet damit

CH₃OH + H₂O → CO₂ + 3 H₂

Eine etwas höhere Temperatur und ein niedrigerer Druck im Vergleich zum Syntheseprozess tragen dazu bei, dass sich das Reaktionsgleichgewicht in Richtung Wasserstoff statt Methanol verschiebt. Eine vollständige Umwandlung des Methanols ist jedoch nicht erforderlich, da das nicht umgewandelte Methanol später vom erzeugten Wasserstoff abgetrennt wird, z.B. in einer Druckwechseladsorption, und anschließend oxidiert wird, um Wärme für die Reformierungsreaktion bereitzustellen.

Um so viel Wasserstoff wie nötig zu erzeugen, ist es von Vorteil, ein nahezu stöchiometrisches Gemisch aus Methanol und Wasser zu reformieren. Bei der Methanolreformierung in kleinem oder mobilen Maßstab kann es von Vorteil sein, das gewünschte Gemisch aus Methanol und Wasser anzuliefern und/oder zu lagern, anstatt es auf Abruf zu produzieren, da das Mischen den Prozess komplexer macht und das Gemisch vorteilhafte Bedingungen für die Lagerung aufweist, wie etwa einen sehr niedrigen Gefrierpunkt. Ein weiterer Vorteil ist beispielsweise auch die Unterdrückung von Algenwachstum und damit sogenanntem Biofouling. Das vorgemischte Ausgangsmaterial wird normalerweise durch Mischen von hochreinem Methanol mit hochreinem Wasser hergestellt.

Aufgrund der ähnlichen Reaktionsbedingungen und Katalysatoren entstehen beim Methanolreforming und bei der Methanolsynthese ähnliche Verunreinigungen wie Ameisensäure. Daher sollten die in Methanolreformern verwendeten Katalysatoren gegenüber den typischen Verunreinigungen, die bei der Methanolsynthese entstehen, einigermaßen tolerant sein.

Die Reinigung des synthetisierten Methanols (Rohmethanol) kann eine beträchtliche Wärmemenge erfordern, und die Destillationskolonnen weisen schlechte dynamische Eigenschaften auf, was die Gesamtdynamik des Prozesses verschlechtert und möglicherweise den Lastbereich des Prozesses einschränkt.

Die Produktion von grünem Methanol leidet unter hohen Energiepreisen in Zeiten geringer erneuerbarer Produktion und geringer Verfügbarkeit von Wärme (Dampf) am jeweiligen Produktionsstandort für die Methanolreinigung. Der Bedarf an einem dynamischen Betrieb ist in letzter Zeit stark gestiegen. Neben dem Ersatz von fossilem Methanol in bestehenden Anwendungsfällen wird erneuerbares Methanol auch als neuartiger Brennstoff diskutiert, z.B. für die Verwendung in Methanolreformern. Solche neuen Verwendungszwecke sind mit neuen Reinheitsanforderungen verbunden.

Wie bereits zuvor erwähnt, werden vorliegend ein Verfahren und eine Anlage zur Herstellung eines Gemischs, das Wasser und Methanol enthält, aus Kohlendioxid und Wasserstoff vor, wobei dieses Gemisch gespeichert und als Ausgangsmaterial für die Methanolreformierung verwendet werden kann. Das Gemisch kann aus dem Reaktionsprodukt der direkten Hydrierung von Kohlendioxid mit minimaler bis gar keiner Reinigung und optionaler Zugabe von kleinen Mengen Wasser hergestellt werden, um das gewünschte Verhältnis von Wasser zu Methanol zu erreichen. Auf diese Weise werden die Nachteile des Standes der Technik überwunden.

In Figur 1 ist eine Ausgestaltung eines vorgeschlagenen Verfahrens schematisch veranschaulicht und insgesamt mit 100 bezeichnet.

Hier vorgeschlagene Ausgestaltungen können auch mit anderen typischen Konfigurationen, die im Stand der Technik bekannt sind, realisiert werden, wie z.B. mit unterschiedlichen Anordnungen von Abscheidern, zusätzlicher Wärmeintegration, unterschiedlichen Verdichteranordnungen usw.

Dem Verfahren 100 werden Wasserstoff H2 und Kohlendioxid CO2 in einem ersten und zweiten Einsatzstrom 1, 2 zugeführt. Nach einer Vereinigung der Einsatzströme 1, 2 und deren Verdichtung in einem Verdichter C1 wird ein entsprechend erhaltener Stoffstrom 3 mit einem unten erläuterten, in einem Verdichter C2 verdichteten Rückführstrom 18 zu einem Sammelstrom 4 vereinigt. Der Sammelstrom 4 wird in einem Wärmetauscher E1 gegen aus einem Reaktor R1 abströmendes Prozessgas 7 erwärmt, wobei ein hier mit 5 bezeichneter Stoffstrom erhalten wird. Stoffstrom 5 wird durch einen Wärmetauscher E2 geführt und dann, mit 6 bezeichnet, zur Umsetzung des Wasserstoffs und Kohlendioxids zu Methanol, dem Reaktor R1 zugeführt.

Das erhaltene Prozessgas 7 umfasst nicht umgesetzten Wasserstoff, nicht umgesetztes Kohlendioxid, Wasser und Methanol sowie Nebenprodukte wie Ester, Ether, Aldehyde und Ketone. Nach Kühlung in dem Wärmetauscher E1 wird das Prozessgas, ggf. mit einem kondensierten Anteil, das nun mit 8 bezeichnet ist, durch einen Wärmetauscher E3 geführt. Ein entsprechend erhaltener Stoffstrom 9 wird in einen Separator S1 eingespeist, aus dessen Sumpf ein flüssiges Gemisch 10 abgezogen werden kann, und von dessen Kopf ein gasförmiges Gemisch 16 von im Wesentlichen Wasserstoff und Kohlendioxid abgezogen werden kann. Letzteres Gemisch 16 kann nach Abzweigen eines Ausschleusstroms 17 mittels des Verdichters C2 zu dem bereits erwähnten Rückführstrom 18 verdichtet werden. Das Gemisch 10 umfasst neben Wasser und Methanol noch einen Anteil von Wasserstoff und Kohlendioxid sowie der genannten Nebenprodukte in gelöster bzw. dispergierter Form.

Das flüssige Gemisch 10 kann zur weiteren Aufreinigung unter Erhalt eines Stoffstroms 11 an einem Ventil V1 entspannt werden, wobei Stoffstrom 11 in einen Separator S2 eingespeist wird. Aus dem Sumpf des Separators kann Rohmethanol 12 mit einem Gehalt von Wasser und Methanol in Summe von typischerweise mehr als 99% auf Gewichts- und Molbasis abgezogen werden kann. Eine Gasfraktion 15, die vom Kopf des Separators abgezogen wird, umfasst zumindest den überwiegenden Teil der Nebenprodukte und Kohlendioxid.

Unter Entspannung an einem Ventil V2 wird ein Stoffstrom 13 gebildet, der einer optionalen Konditionierung 110 unterworfen werden kann. Eine Konditionierung kann auch unterbleiben. Dennoch ist in jedem Fall ein ohne oder mit Konditionierung gebildeter Stoffstrom in Figur 1 mit 14 bezeichnet. Dieser wird nun einem oder mehreren Verfahrensschritten 120 zur Reformierung von Methanol unterworfen. Eine Speicherung bzw. ein Transport können vorgesehen sein und sind nur der Übersichtlichkeit halber nicht veranschaulicht.

Generell kann die optionale Konditionierung eine Zugabe von Wasser und/oder eine Entfernung von Verunreinigungen umfassen. Die Konditionierung kann beispielsweise die Zugabe von Wasser in Form eines Stoffstroms 20 umfassen, um auf diese Weise das gewünschte Verhältnis von Methanol zu Wasser zu erreichen. Das Verhältnis von Wasser zu Methanol kann zwischen 20:80 und 80:40 liegen, insbesondere von 30:70 bis 45:55 und weiter insbesondere von 30:70 bis 45:55. Das stöchiometrische Verhältnis beträgt ca. 36:64.

Die Entfernung von leichtflüchtigen Verbindungen wie Kohlendioxid, Estern, Ethern, Aldehyden und Ketonen etc. kann ebenfalls Teil der Konditionierung 110 sein. Sie kann beispielsweise in einer Rektifikationskolonne mit Sumpfaufkocher erfolgen, wobei deutlich weniger, insbesondere um mehr als 50% weniger oder um mehr als 80% weniger Energieaufwand als für eine Reinigung im Stand der Technik erforderlich ist.

Eine Entfernung von leichten/flüchtigen Verbindungen wie Kohlendioxid und Methylformiat kann beispielsweise in einem Stripper oder einer Stripperkolonne erfolgen, die Teil der Konditionierung ist. Ein entsprechender Stripper kann mit Strippgas 21 betrieben werden.

Bei der Entfernung von Verbindungen aus Stoffstrom 13 kann ein gebildeter Abgasstrom 22 oder ein Teil davon stromaufwärts des Reaktors R1 rezykliert oder zur Wärmeerzeugung oxidiert werden.

Eine Möglichkeit, einen Stripper zu betreiben, besteht darin, Wasserstoff zu verwenden und den Abgasstrom 22 in einen Strom stromaufwärts des Reaktors R1 zu recyceln. Eine andere Möglichkeit besteht darin, den Stripper mit Stickstoff zu betreiben und den Abgasstrom 22 zu verwerfen oder zu verbrennen.

Nach der Konditionierung 110 erhaltene Endprodukt enthält typischerweise mehr als 99% Methanol und Wasser in Summe, insbesondere mehr als 99,95%.

Insgesamt ermöglicht das vorgeschlagene Verfahren eine höhere Energieeffizienz durch einen geringeren Wärmebedarf bei der Produktaufbereitung, eine verbesserte Prozessdynamik des Prozesses sowie einen erweiterten Lastbereich des Prozesses bei einer geringeren Gesamtprozesskomplexität.

## Patentansprüche

1. Verfahren (100) zur Bereitstellung und Reformierung von Methanol, bei dem ein Wasser und Methanol enthaltendes Reformierungseinsatzgemisch (14) bereitgestellt und einem oder mehreren Reformierungsschritten (120) unterworfen wird, wobei die Bereitstellung des Reformierungseinsatzgemischs (14) einen oder mehrere Methanolsyntheseschritte umfasst, in dem oder denen ein Wasser und Methanol enthaltendes Methanolsyntheseproduktgemisch (7) gebildet wird, wobei das in dem Methanolsyntheseproduktgemisch (7) enthaltene Wasser oder ein Teil hiervon und das in dem Methanolsyntheseproduktgemisch (7) enthaltene Methanol oder ein Teil hiervon ohne eine Trennung voneinander in das Reformierungseinsatzgemisch (14) überführt werden.

2. Verfahren (100) nach Anspruch 1, das eine Aufbereitung des Methanolsyntheseproduktgemischs (7) oder eines Teils hiervon umfasst, bei der ein Zwischengemisch (12) gebildet wird, das einen Anteil von mehr als 99% Wasser und Methanol in Summe enthält.

3. Vefahren (100) nach Anspruch 2, das eine Konditionierung (110) des Zwischengemischs (12) oder eines Teils hiervon umfasst.

4. Verfahren (100) nach Anspruch 3, bei dem die Konditionierung (110) eine Einstellung eines Zielverhältnisses von Wasser zu Methanol durch eine Zuspeisung von Wasser (20), wobei das Zielverhältnis von Wasser zu Methanol zwischen 20:80 und 80:40, zwischen 30:70 und 45:55 oder zwischen 30:70 und 45:55 Molanteilen beträgt.

5. Verfahren (100) nach einem der Ansprüche 3 oder 4, bei dem die Konditionierung (110) eine Entfernung einer oder mehrerer leichtflüchtiger Verbindungen umfasst.

6. Verfahren (100) nach Anspruch 5, bei dem die eine oder die mehreren leichtflüchtigen Verbindungen aus Kohlendioxid, einem oder mehreren Estern, einem oder mehreren Ethern, einem oder mehreren Aldehyden und/oder einem oder mehreren Ketonen ausgewählt ist oder sind.

7. Verfahren (100) nach Anspruch 5 oder 6, bei dem die Entfernung der einen oder der mehreren leichtflüchtigen Verbindungen unter Einsatz einer Rektifikation durchgeführt wird.

8. Verfahren (100) nach einem der Ansprüche 5 bis 7, bei dem die Entfernung der einen oder der mehreren leichtflüchtigen Verbindungen unter Einsatz einer Strippung durchgeführt wird.

9. Verfahren (100) nach Anspruchs, bei dem die Strippung unter Verwendung von Strippgas (21) und unter Erhalt eines Strippabgases (22) durchgeführt wird, wobei das Strippgas (21) Wasserstoff enthält und das Strippabgas (22) oder ein Teil hiervon in das Verfahren (100) zurückgeführt wird und/oder das Strippgas (21) Stickstoff enthält und das Strippabgas (22) oder ein Teil hiervon aus dem Verfahren (100) ausgeleitet wird.

10. Verfahren (100) nach einem der Ansprüche 2 bis 9, bei dem die Aufbereitung nacheinander eine erste Trennung auf einem ersten Druckniveau, eine Entspannung auf ein zweites Druckniveau und eine zweite Trennung auf dem zweiten Druckniveau umfasst.

11. Verfahren zur Bereitstellung eines Reformierungseinsatzgemischs (14), das umfasst, einen oder mehrere Methanolsyntheseschritte durchzuführen, in dem oder denen ein Wasser und Methanol enthaltendes Methanolsyntheseproduktgemisch (7) gebildet wird, wobei das in dem Methanolsyntheseproduktgemisch (7) enthaltene Wasser oder ein Teil hiervon und das in dem Methanolsyntheseproduktgemisch enthaltene Methanol oder ein Teil hiervon ohne Trennung voneinander in das Reformierungseinsatzgemisch (14) überführt werden und das Reformierungseinsatzgemisch (14) dem Verfahren entzogen und einer Speicherung und/oder einem Transport unterworfen wird.

12. Anlage zur Bereitstellung und Reformierung von Methanol, die dafür eingerichtet ist, ein Wasser und Methanol enthaltendes Reformierungseinsatzgemisch (14) bereitzustellen und einem oder mehreren Reformierungsschritten (120) zu unterwerfen, wobei die Anlage dafür eingerichtet ist, zur Bereitstellung des Reformierungseinsatzgemischs (14) einen oder mehrere Methanolsyntheseschritte durchzuführen, in dem oder denen ein Wasser und Methanol enthaltendes Methanolsyntheseproduktgemisch (7) gebildet wird, und wobei die Anlage dafür eingerichtet ist, wobei das in dem Methanolsyntheseproduktgemisch (7) enthaltene Wasser oder einen Teil hiervon und das in dem Methanolsyntheseproduktgemisch enthaltene Methanol oder einen Teil hiervon ohne eine Trennung voneinander in das Reformierungseinsatzgemisch (14) zu überführen.

13. Anlage zur Bereitstellung eines Reformierungseinsatzgemischs, die dafür eingerichtet ist, einen oder mehrere Methanolsyntheseschritte durchzuführen, in dem oder denen ein Wasser und Methanol enthaltendes Methanolsyntheseproduktgemisch (7) gebildet wird, wobei die Anlage dafür eingerichtet ist, das in dem Methanolsyntheseproduktgemisch (7) enthaltene Wasser oder einen Teil hiervon und das in dem Methanolsyntheseproduktgemisch enthaltene Methanol oder einen Teil hiervon ohne eine Trennung voneinander in das Reformierungseinsatzgemisch (14) zu überführen und das Reformierungseinsatzgemisch für eine Speicherung und/oder einen Transport aus der Anlage auszuschleusen.

14. Anlage nach Anspruch 12 oder 13, die zur Durchführung eines Verfahrens nach einem der Ansprüche 1 bis 11 eingerichtet ist.
